Europäisches Patentamt

European Patent Office

Office européen des brevets

(19)

(11) EP 1 391 730 A1

## (12) EUROPEAN PATENT APPLICATION
published in accordance with Art. 158(3) EPC

(43) Date of publication:
**25.02.2004 Bulletin 2004/09**

(21) Application number: **02722906.1**

(22) Date of filing: **30.04.2002**

(51) Int Cl.⁷: **G01N 33/544**, A61M 1/36,
A61K 9/00, A61K 38/28

(86) International application number:
**PCT/JP2002/004332**

(87) International publication number:
**WO 2002/090990 (14.11.2002 Gazette 2002/46)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **09.05.2001 JP 2001139280**

(71) Applicants:
• **KYOWA MEDEX CO., LTD.**
**Tokyo 104 (JP)**
• **A School Corporation Kansai University**
**Suita-shi, Osaka 564-8680 (JP)**

(72) Inventors:
• **MIYATA, Takashi**
**Suita-shi, Osaka 565-0872 (JP)**
• **URAGAMI, Tadashi**
**Mino-shi, Osaka 562-0022 (JP)**
• **FUKUI, Masanori,c/o Head Office**
**Chuo-ku, Tokyo 104-0042 (JP)**

(74) Representative: **Tanner, James Percival**
**D. Young & Co,**
**21 New Fetter Lane**
**London EC4A 1DA (GB)**

(54) **BIOPOLYMER-CONTAINING GEL**

(57) Disclosed are a gel in which two or more ligands which are biomacromolecules are grafted, a process for producing the gel, a method for measuring a specific molecule in a sample using the gel, an apparatus, a measurement kit and a pharmaceutical composition comprising the gel, and a diagnostic method for various diseases using the measurement kit.

Fig. 4

Imprinted molecule-unremoved gel

AFP-imprinted gel

**Description**

Technical Field

The present invention relates to gels which undergo structural changes induced by specific recognition of biomacromolecules such as proteins, DNA and polysaccharides, a process for producing the gels, and use of the gels.

Background Art

Gels exhibit phase transitions in response to changes in the surrounding physicochemical conditions, for example, pH, temperature and solvent composition. As a result, the swelling ratio changes, which induces great changes in volume.

Gels so far reported include those which undergo volume changes in response to a change in the affinity of polymer chains constituting a gel for a solvent, a change in charged groups in polymer chains, etc. [Phys. Rev. Lett., 40, 820 (1978); Phys. Rev. Lett., 45, 1636 (1980); Sci. Am., 244, 123 (1981); Responsive Gels, Volume Transitions I & II; Adv. Polym. Sci., 109 & 110, Springer, Berlin (1993)]. As described above, volume changes of gels are in most cases induced by changes in the surrounding physicochemical conditions. Known examples of gels which undergo structural changes induced by recognition of specific molecules are glucose-responsive gels [Macromol. Chem. Phys., 197, 1135 (1996); J. Am. Chem. Soc., 120, 12694 (1998)].

On the other hand, it is known that gels recognizing specific molecules can be produced by the molecular imprinting technique in which a specific molecule is used as a template and a substance interacting with the molecule is chemically immobilized in a gel [Angew. Chem., Int. Ed. Engl., 34, 1812 (1995)]. So far, low molecular weight compounds have been used as the substances which interact with specific molecules and which are immobilized in gels (hereinafter, the substance interacting with a molecule is referred to as a ligand).

In producing gels precisely recognizing biomacromolecules such as proteins having high molecular weights, it is difficult to properly arrange low molecular weight ligands recognizing different parts of a biomacromolecule in a gel in order to incorporate the specific molecule into the gel. U.S. Patent No. 5,801,221 discloses gels prepared by introducing ligands utilizing the molecular recognizing ability of antibodies, but introduction of antibodies per se into gels is not disclosed. This patent discloses molecular imprinted gels which are capable of changing the molecular recognizing or binding ability by utilizing the volume phase transition in response to external stimuli.

There are reports on a gel into which an antibody has been introduced [Macromolecules, 32, 2082 (1999) ; Nature, 399, 766 (1999)]. This gel is prepared by chemically modifying an antigen and an antibody, and then forming a gel network from the antigen-antibody complex and another hydrophilic monomer by applying the antigen-antibody binding at crosslinking points. The gel contains the antigen-antibody binding therein, and therefore, when an antigen or an antibody is added to the gel, the gel swells but does not shrink. The gel is not capable of structural changes of molecules other than those at crosslinking points.

For the selective and sensitive detection or determination of substances contained in trace amounts in mixtures, immunoassays such as radioimmunoassay (RIA) and enzyme immunoassay (EIA) utilizing antigen-antibody reactions [Enzyme-linked Immunosorbent Assay (ELISA), Igaku Shoin (1976)], etc. have been proposed.

Immunoassays are methods of determining the amount of an antibody or an antigen by using an antigen or an antibody labeled with an enzyme, a radioisotope or the like, and include, for example, competitive assay and sandwich assay [Immunology Illustrated, 5th ed. (Nankodo)].

In the sandwich assay, a primary antibody is immobilized on a solid phase support and an antigen to be determined is trapped, followed by reaction with a labeled secondary antibody.

Further, antibody-lectin enzyme immunoassay has been recently reported as a method for measuring glycoproteins [Biophysical Chemistry, 35, 45 (1991)]. This method, in which lectin is used instead of a labeled antibody, is expected to be useful for detecting α-fetoprotein (AFP) which is a marker indicating the malignancy of hepatoma.

However, detection by sandwich assay is difficult when the binding constants between a primary antibody and an antigen and between a secondary antibody or lectin and the antigen are greatly different from each other and when the optimum conditions for their bindings are different. Further, immunoassay is disadvantageous in that nonspecific adsorption of a protein on an immobilization support lowers the measurement accuracy and that two or more steps are required for measurement.

A need exists for gels recognizing biomolecules, which are expected to be utilizable in a wide range of fields such as diagnosis and other medical fields.

There also exists a need for a less costly and less complex assay method having higher sensitivity, accuracy and specificity as compared with enzyme immunoassays currently employed in medical fields.

Disclosure of the Invention

An object of the present invention is to provide a gel capable of recognizing a biomolecule with high sensitivity, accuracy and specificity which is repeatedly usable by a simple procedure. Another object of the present invention is to provide a process for producing the gel capable of recognizing a biomolecule with high sensitivity, accuracy and specificity which is repeatedly usable by a simple procedure, a method for measuring a specific molecule in a sample using the gel, an apparatus comprising the gel, a measurement kit comprising the gel, a diagnostic method for various diseases using the measurement kit, and a pharmaceutical composition comprising the gel.

The present invention relates to the following (1) to (15).

(1) A gel in which two or more ligands which are biomacromolecules are grafted.

(2) The gel according to the above (1) which undergoes a structural change induced by binding of a specific molecule which binds to the ligands.

(3) The gel according to the above (1) or (2) which is produced by molecular imprinting using a specific molecule as a template and in which two or more ligands for the specific molecule are grafted and the specific molecule is not contained (hereinafter referred to as a molecular imprinted gel).

(4) The gel according to the above (1) or (2) which is produced by molecular imprinting using a specific molecule as a template and in which two or more ligands for the specific molecule are grafted and the specific molecule is contained (hereinafter referred to as an imprinted molecule-unremoved gel).

(5) The gel according to the above (1) or (2) which is produced without utilizing molecular imprinting using a specific molecule as a template and in which two or more ligands for the specific molecule are grafted (hereinafter referred to as a nonimprinted gel).

(6) The gel according to any one of the above (1) to (3) which shrinks by the action of a specific molecule.

(7) The gel according to any one of the above (1), (2), (4) and (5) which swells by the action of a specific molecule.

(8) The gel according to any one of the above (1) to (7), wherein the biomacromolecule is selected from the group consisting of a protein, an antigen, an antibody, the complementarity determining region of an antibody, lectin, DNA, RNA, a lipid, a polysaccharide and a complex thereof.

(9) A process for producing a molecular imprinted gel which comprises the step of chemically immobilizing in a gel two or more ligands for a specific molecule which are bound to the specific molecule as a template, and the step of removing the specific molecule from the gel.

(10) A process for producing an imprinted molecule-unremoved gel which comprises the step of chemically immobilizing in a gel two or more ligands for a specific molecule which are bound to the specific molecule as a template.

(11) A process for producing a nonimprinted gel which comprises the step of chemically immobilizing in a gel two or more ligands for a specific molecule without using the specific molecule as a template.

(12) A method for measuring a specific molecule in a sample which is characterized by use of the gel according to any one of the above (1) to (8).

(13) An apparatus comprising the gel according to any one of the above (1) to (8).

(14) A measurement kit comprising the gel according to any one of the above (1) to (8).

(15) A pharmaceutical composition comprising the gel according to any one of the above (1) to (8).

(16) A diagnostic method for various diseases using the measurement kit according to the above (14).

1. Process for Producing Molecular Imprinted Gel, Imprinted Molecule-Unremoved Gel and Nonimprinted Gel

The molecular imprinted gel of the present invention is a gel which is produced by molecular imprinting and in which two or more ligands are grafted and a specific molecule interacting with the ligands is not contained. That is, the gel is a gel produced by chemically immobilizing in a gel two or more ligands for a specific molecule which are bound to the specific molecule as a template, and then removing the specific molecule.

The imprinted molecule-unremoved gel of the present invention is a gel which is produced by molecular imprinting and in which two or more ligands are grafted and a specific molecule interacting with the ligands is contained. That is, the gel is a gel produced by chemically immobilizing in a gel two or more ligands for a specific molecule which are bound to the specific molecule as a template, without removing the specific molecule.

The nonimprinted gel of the present invention is a gel which is produced without utilizing molecular imprinting and in which two or more ligands are grafted. That is, the gel is a gel produced by chemically immobilizing in a gel two or more ligands for a specific molecule without using the specific molecule as a template.

The molecular imprinted gel and imprinted molecule-unremoved gel of the present invention can be produced, for example, by molecular imprinting [Angew. Chem., Int. Ed. Engl., 34, 1812 (1995)] in which a specific molecule is used as a template and ligands for the specific molecule are chemically immobilized in a gel.

The specific molecules are not particularly limited and include high molecular weight substances such as proteins, DNA, RNA, antigens, antibodies and polysaccharides, and low molecular weight substances such as metal ions, as well as molecules with the molecular structure altered. Examples of the molecules with the molecular structure altered include mutant molecules in which a part of a sugar chain of a glycoprotein is altered, and molecular complexes in which molecules interact with each other such as a protein-protein complex, a protein-DNA complex, a DNA-RNA complex and a metal complex. An example of the mutant molecule in which a part of a sugar chain of a glycoprotein is altered is $\alpha$-fetoprotein (hereinafter referred to as AFP) which is known as a marker for hepatic diseases. There is no specific restriction as to the molecular weight of the specific molecules to be used, and those of any molecular weight can be employed.

As the ligand of the present invention, any biomacromolecules interacting with the specific molecules can be used. Suitable ligands include proteins, DNA, RNA, antigens, antibodies, the complementarity determining region (CDR) of antibodies, lectin, lipids, polysaccharides and complexes thereof. Examples of the complexes include fusion proteins of different proteins, fusion antibodies of protein and antibody, and bispecific antibodies having two different binding regions and capable of recognizing two different target molecules. There is no specific restriction as to the molecular weight of the ligands to be used, and those of any molecular weight can be employed.

Structural changes of specific molecules can be examined by using a combination of at least two ligands. As the combination of two or more ligands, the above-described ligands may be used in combination, or a complex of ligands may be used singly. Two or more ligands may be a combination of different ligands for a single biomacromolecule, a combination of ligands for two or more biomacromolecules, and the like.

Specifically, when the specific molecule is AFP which is a glycoprotein, an example of a suitable combination of ligands for AFP is a combination of an antibody recognizing AFP and concanavalin A (Con. A) which is lectin binding to a sugar chain.

The molecular imprinted gel of the present invention can be produced by molecular imprinting, for example, a process which comprises (1) the step of chemically immobilizing in a gel two or more ligands for a specific molecule which are bound to the specific molecule as a template, and (2) the step of removing the specific molecule from the gel. In this process, the imprinted molecule-unremoved gel is produced in the step of (1). Specifically, the molecular imprinted gel of the present invention can be produced, for example, in the following manner.

A ligand which is a biomacromolecule recognizing a specific molecule is subjected to reaction with a monomer or a synthetic polymer prepared in advance by polymerization of monomers (hereinafter referred to as a polymer) as a material for a gel to prepare a ligand-introduced monomer or a ligand-introduced polymer. Subsequently, the ligand-introduced monomers are polymerized and crosslinked, with the specific molecule bound to the ligand, or the ligand-introduced polymers are crosslinked, with the specific molecule bound to the ligand, to form a polymer network, whereby the imprinted molecule-unremoved gel is produced. Then, the molecular imprinted gel can be produced by removing the specific molecule from the gel.

The ligand-introduced monomer or ligand-introduced polymer can be prepared by reaction of a reactive functional group existing in the monomer or polymer with a reactive functional group existing in the biomacromolecule which is the ligand.

As the monomer, any monomers having a reactive functional group which reacts with the ligand and taking a tertiary structure by polymerization reaction can be used. For example, monomers having a reactive functional group which reacts with the ligand and a reactive functional group for synthesizing a gel can be used. The reactive functional group which reacts with the ligand and the reactive functional group for synthesizing a gel may be the same or different. There is no specific restriction as to the molecular weight of the monomers to be used, and those of any molecular weight can be employed.

Among the monomers having a reactive functional group, vinyl monomers having a reactive functional group are preferred. Examples of the reactive functional groups include vinyl group, epoxy group, allyl group, hydroxyl group, carboxyl group, amino group, aldehyde group, phosphate group, cyano group and sulfhydryl group.

Examples of the vinyl monomers having a reactive functional group include acrylic acid, methacrylic acid, vinyl acetate, vinylamine and their derivatives, 2-hydroxyethyl methacrylate and acrylonitrile.

When the reactive functional group is carboxyl group, esters of the above vinyl monomers may also be used. Examples of the esters include methyl acrylate, ethyl acrylate, methyl methacrylate, ethyl methacrylate, N-succinimidyl acrylate and N-succinimidyl methacrylate, among which N-succinimidyl acrylate and N-succinimidyl methacrylate are preferably used.

As the polymer, any polymers having a reactive functional group which reacts with the ligand and forming a polymer network by crosslinking reaction of polymers can be used. Preferred polymers are those having a reactive functional group which reacts with the ligand and a reactive functional group for synthesizing a gel. The reactive functional group which reacts with the ligand and the reactive functional group for synthesizing a gel may be the same or different. Vinyl polymers having a reactive functional group are preferably used. There is no specific restriction as to the molecular weight of the polymers to be used, and those of any molecular weight can be employed.

Suitable polymers include polymers of the above-described monomers, condensation polymers such as polyester polymer, natural polymers such as cellulose and chitin and derivatives thereof, polystyrene resin, polyurethane resin, polyamide resin, polyimide resin and epoxy resin. The polymer network may be formed from different kinds of polymers.

As the vinyl polymer having a reactive functional group, any vinyl polymers having a reactive functional group such as hydroxyl group, carboxyl group, amino group or phosphate group can be used. Suitable vinyl polymers include polyvinyl alcohol and poly(2-hydroxyethyl methacrylate) having hydroxyl group, polyacrylic acid and polymethacrylic acid having carboxyl group, polyvinylamine having amino group, and derivatives of these polymers. When the reactive functional group is carboxyl group, esters of the above vinyl polymers may also be used. Examples of the esters include methyl esters, ethyl esters and active esters (e.g., N-succinimidyl ester).

In cases where a monomer or a polymer having a functional group with low reactivity is used, the functional group may be converted to the above-described reactive functional group by chemical modification prior to the reaction with the ligand which is a biomacromolecule.

An example of the process for producing the vinyl monomer containing the introduced ligand is given below. One of the amino groups of a diamino compound having two or more amino groups is reacted with phosphate group of DNA which is the ligand. Then, the other amino group of the diamino compound is reacted with a vinyl monomer having carboxyl group or a vinyl monomer having ester group such as succinimidyl acrylate, whereby the DNA which is the ligand can be introduced into the vinyl monomer having the reactive functional group.

The imprinted molecule-unremoved gel of the present invention can be produced by binding the specific molecule for the ligand to the above ligand-introduced monomer or ligand-introduced polymer, and then forming the polymer network in the presence of a crosslinking agent and a reaction initiator or in the presence of a crosslinking agent, a reaction initiator and a reaction accelerator.

As the crosslinking agent for the ligand-introduced monomer, any multifunctional monomers can be used. For example, divinyl crosslinking agents, preferably, diacrylate crosslinking agents can be used.

As the crosslinking agent for the ligand-introduced polymer, any multifunctional compounds having two or more reactive functional groups can be used. Suitable crosslinking agents include dialcohol, trialcohol, dicarboxylic acid, tricarboxylic acid, diamine, triamine, diisocyanate, triisocyanate, diglycidyl, triglycidyl, dialdehyde, trialdehyde, divinyl, trivinyl, dithiol and trithiol.

In the production of the gel by polymerization of the ligand-introduced monomers, the gel can be produced not only by polymerization of the ligand-introduced monomer and the crosslinking agent but also by polymerization of three or more components, i.e., the ligand-introduced monomer, at least one kind of monomer and the crosslinking agent. As the monomer, any of the above-described monomers can be used, and vinyl monomers are preferably used.

As the reaction initiator, any radical polymerization initiators activating vinyl group and forming a radical can be used. Suitable reaction initiators include benzoyl peroxide, ammonium persulfate (APS) and azobisisobutyronitrile. As the reaction accelerator, any substances accelerating the formation of polymer network can be used. For example, N,N,N',N'-tetramethylethylenediamine (TEMED) can be used.

After the above reaction, the unreacted monomer or unreacted polymer is removed from the gel, whereby the imprinted molecule-unremoved gel of the present invention can be obtained.

Removal of the unreacted monomer or unreacted polymer from the gel can be carried out, for example, by washing in buffer having pH around neutrality. As the buffer, phosphate buffer and the like can be used.

The molecular imprinted gel of the present invention can be obtained by removing the specific molecule from the imprinted molecule-unremoved gel obtained above. Removal of the specific molecule from the gel can be carried out, for example, by washing with buffer having pH suitable for breaking the intermolecular bond between the ligand and the specific molecule, by using ultrasonic waves, or by using ultrasonic waves in buffer having pH around neutrality. As the buffer, phosphate-citrate buffer and the like can be used.

The nonimprinted gel of the present invention can be produced by a process which comprises the step of grafting two or more ligands in a gel without utilizing molecular imprinting, that is, the step of chemically immobilizing in a gel two or more ligands for a specific molecule without using the specific molecule as a template.

The thus prepared imprinted molecule-unremoved gel, molecular imprinted gel and nonimprinted gel can be used in the method for measuring a specific component in a sample, apparatus, measurement kit, pharmaceutical composition and diagnostic method for various diseases described hereinbelow.

Shown below are the processes for producing the molecular imprinted gel and nonimprinted gel wherein the specific molecule is AFP which is known as a marker for hepatic diseases.

Process for Producing Molecular Imprinted Gel Recognizing AFP

AFP is a glycoprotein. In the molecular imprinted gel recognizing AFP, an antibody against AFP (hereinafter referred to as an anti-AFP antibody) and concanavalin A (hereinafter referred to as Con. A) which is lectin binding to a sugar chain can be used as the ligands.

First an anti-AFP antibody and Con. A are chemically modified. That is, N-succinimidyl acrylate is reacted with the amino group of the anti-AFP antibody and with that of Con. A to synthesize an antibody-introduced vinyl monomer and a Con. A-introduced vinyl monomer.

Then, AFP as the specific molecule which is sandwiched between the antibody-introduced vinyl monomer and the Con. A-introduced vinyl monomer is subjected to reaction with a hydrophilic vinyl monomer such as acrylamide and a divinyl crosslinking agent in the presence of a reaction initiator in a glass tube to form a polymer network.

After the reaction, the gel is washed with phosphate buffer (pH 7.4) to remove the unreacted monomers. Subsequently, AFP as the specific molecule is removed by washing the gel with phosphate-citrate buffer (pH 4.0).

The molecular imprinted gel recognizing AFP (hereinafter referred to as AFP-imprinted gel) can be produced by the above process. The above AFP-imprinted gel recognizes biomacromolecules at multiple points. The AFP-imprinted gel gives different responses according to the type of substance subjected to reaction with the gel. In cases where the substance is one recognizing both Con. A and the anti-AFP antibody (e.g., AFP), the gel shrinks. In cases where the substance is one recognizing either Con. A or the anti-AFP antibody (e.g., sugar chain-mutated AFP which is mutant AFP having mutation only at a part of the sugar chain of AFP, and chicken ovalbumin), the gel swells. In cases where the substance is one recognizing neither Con. A nor the anti-AFP antibody [e.g., bovine serum albumin (BSA)], the gel neither shrinks nor swells, causing no volume change.

Process for Producing AFP-Nonimprinted Gel

The AFP-nonimprinted gel is a gel in which two or more ligands for AFP are grafted and which is produced without utilizing molecular imprinting. As the ligands for AFP, a combination of an anti-AFP antibody and Con. A and the like can be used.

Similarly to the production of AFP-imprinted gel, an anti-AFP antibody and Con. A are chemically modified to synthesize an antibody-introduced vinyl monomer and a Con. A-introduced vinyl monomer.

Then, in the absence of AFP as the specific molecule, the antibody-introduced vinyl monomer and the Con. A-introduced vinyl monomer are subjected to reaction with a hydrophilic vinyl monomer such as acrylamide and a divinyl crosslinking agent in the presence of a reaction initiator in a glass tube to form a polymer network. The gel formed by the reaction is washed with phosphate buffer or the like.

2. Method for Measuring a Specific Molecule in a Sample Using the Gel of the Present Invention

When the molecular-imprinted gel is brought into contact with a sample containing a specific molecule, the specific molecule and the ligands form a complex, which causes the gel to shrink.

When the imprinted molecule-unremoved gel is brought into contact with a sample containing a specific molecule, the complex already formed from the specific molecule and the ligands in the gel (partially) disappears, which causes the gel to swell.

When the nonimprinted gel is brought into contact with a sample containing a specific molecule, the number of specific molecules bound in the gel increases, which causes the gel to swell.

Accordingly, qualitative analysis of a substance contained in a sample can be carried out by observing the gel after an exposure to the sample. The volume change of the gel may be directly observed with a microscope or the like, but is preferably analyzed based on the change in absorption spectrum after irradiation of the gel that shrank or swelled with laser beam, infrared ray, visible light, ultraviolet ray, or the like. The volume change of the gel due to the shrinking or swelling of the gel is dependent upon the amount of a specific molecule in a sample. Therefore, it is also possible to quantitatively determine a specific molecule in a sample by measuring the volume change of the gel due to the shrinking or swelling of the gel using the apparatus described in 4 below.

There is no specific restriction as to the sample containing a specific molecule, so far as it is a sample capable of measurement of a specific molecule using the gel of the present invention. Suitable samples include blood, plasma, serum, cerebrospinal fluid, saliva, amniotic fluid, urine, sweat and pancreatic juice.

Besides the above-described measurement of volume change due to the shrinking or swelling of the gel, the quantitative determination of a specific molecule using the molecular imprinted gel, imprinted molecule-unremoved gel or nonimprinted gel can be carried out also by the following method. That is, a labeling substance is caused to be carried within the gel by covalent bond, electrostatic interaction, hydrogen bond, hydrophobic interaction, or other types of adsorption, and when the gel swells, the labeling substance is released from the gel. By measuring the amount of the labeling substance released from the gel, the amount of the specific molecule can be determined.

Suitable labeling substances include radioisotopes such as $^{125}$I and $^{32}$P, enzymes such as alkaline phosphatase, peroxidase and luciferase, luminescent substances such as acridinium ester and lophine, pigments such as Methylene Blue, couplers such as orthophenylenediamine, and fluorescent substances such as fluorescein isothiocyanate (FITC).

3. Measurement Kit Comprising the Gel of the Present Invention and Diagnostic Method Using It

The molecular imprinted gel, imprinted molecule-unremoved gel or nonimprinted gel of the present invention constitutes a measurement kit useful in a diagnostic method for various diseases as described below. Previous measurement kits are comprised of plural reagents, but the measurement kit of the present invention can be comprised of the molecular imprinted gel, imprinted molecule-unremoved gel or nonimprinted gel produced by the process of the above 1 alone. The molecular imprinted gel of the present invention can be repeatedly used because the binding of ligands to a specific molecule within the gel is a reversible reaction.

The molecular imprinted gel, imprinted molecule-unremoved gel or nonimprinted gel of the present invention recognizes a specific molecule interacting with the ligands and therefore can be used in the diagnosis of a disease in which the specific molecule is concerned.

As described above, the molecular imprinted gel shrinks when a sample contains a molecule identical to the imprinted specific molecule. The imprinted molecule-unremoved gel swells when a sample contains a substance identical to the specific molecule bound in the gel. The nonimprinted gel swells when a sample contains the specific molecule.

According to the diagnostic method using the gel of the present invention, structural changes of a specific molecule can be recognized.

The structural changes of a specific molecule include a change in the tertiary structure of a specific molecule and a change in the partial structure due to an abnormal site in a molecule.

Transcription and translation of various DNAs are regulated by binding of a complex formed by plural proteins to DNA or its dissociation from DNA.

By use of the gel of the present invention, it is possible to detect simply in one step the presence of a binding of DNA to a protein complex, an abnormality in the tertiary structure or partial structure of plural proteins forming a complex, and the like. In particular, an abnormality in the tertiary structure or partial structure of plural proteins forming a complex can be detected simply and accurately.

More specifically, the occurrence of an abnormality in a part of the proteins forming a complex can be detected by use of a gel produced using, as the ligands, antibodies recognizing the tertiary structure of the antigen proteins forming the complex, or antibodies recognizing partial amino acid sequences existing on the surface of the complex.

Further, diagnosis of various diseases can be made by using, as an index, a change in the tertiary structure or partial structure of a protein in a living organism.

The protein in a living organism may be any of the proteins related to diseases or their complexes. Examples of the proteins include α-fetoprotein (AFP) which is a glycoprotein known as a marker for hepatic diseases such as hepatoma and cirrhosis, and prion protein and β-amyloid which are considered to be causal factors for Alzheimer disease. By detection of a change in the tertiary structure or partial structure of such proteins in a living organism, diagnosis of diseases such as hepatoma, cirrhosis and Alzheimer disease can be conducted.

As the ligands for glycoproteins such as AFP, anti-glycoprotein antibodies (e.g., anti-AFP antibody), lectin, etc. are employed.

Prion protein, β-amyloid, etc. are considered to induce a disease through a change in the tertiary structure, not through a change in the amino acid sequence. Therefore, as the ligands for such proteins, antibodies recognizing tertiary structure, antibodies recognizing surface antigens of proteins, etc. are employed.

4. Apparatus and Pharmaceutical Composition Comprising the Gel of the Present Invention

The gels of the present invention can be used in an apparatus based on their properties described above, that is, the shrinking or swelling of the gels in response to a specific molecule in a sample.

An example of such an apparatus is one in which the gel of the present invention is immobilized on the surface of a minute sensor chip connected with a weigher and a volume change of the gel on the surface of the sensor chip due to shrinking or swelling is indicated as a weight change. When a sample containing a specific molecule is contacted with the surface of the sensor chip, the specific molecule is incorporated into the gel, causing an increase in the weight of the gel, and on the other hand, also causing shrinking or swelling of the gel. The change in the weight of the gel is dependent on the change in the volume of the gel due to shrinking or swelling. This change in the volume of the gel due to shrinking or swelling is dependent on the amount of the specific molecule incorporated into the gel, and the amount of the specific molecule incorporated into the gel is dependent on the amount of the specific molecule contained in the sample. Therefore, the amount of the specific molecule in the sample can be determined by measuring the change in the weight of the gel due to the change in its volume. Similarly, the amount of a specific molecule in a sample can be determined by use of an apparatus in which the sensor chip is connected with a film thickness measurement system instead of the weigher. In this case, the amount of the specific molecule in the sample can be determined by detecting a change in the film thickness in response to the concentration of the specific molecule in the sample. Further, the determination of a specific molecule in a sample can also be carried out by using an apparatus in which the gel of the present invention is connected with an elastic capillary. In this case, the amount of the specific molecule in the sample can be determined by detecting a change in the capillary length in response to the concentration of the specific molecule in the sample.

The gels are applicable to apparatuses for the diagnosis of various diseases described in the above 3 and any other apparatuses capable of comprising the gel of the present invention as a sensor part.

The apparatuses capable of comprising the gel of the present invention as a sensor part include valves, artificial organs, catalytic reaction systems, etc.

Examples of the valves are water supply valves, valves in the production line in a factory and valves in a system for treatment of factory wastes.

For example, the imprinted molecule-unremoved gel of the present invention may be set as a sensor in a water supply valve. In this case, the specific molecule may be any of the harmful substances such as heavy metals and microorganisms that may possibly be contained in city water. As the ligands, antibodies against the specific molecule and the like can be used. The imprinted molecule-unremoved gel is prepared using the ligands-and the specific molecule according to the process of the above 1. This gel set in a water supply valve swells upon exposure to the specific molecule at a concentration beyond a certain degree to cause the valve to be closed, thereby avoiding a supply of water containing a harmful substance.

The gels of the present invention can also be used as organs in a living organism, that is, as artificial organs. For example, artificial pancreas comprising the insulin-containing molecular imprinted gel described below releases insulin by the shrinking of the gel in response to the presence of excess glucose in blood, and thus can be used as artificial pancreas serving a part of the pancreatic function.

It is also possible to create a new catalytic reaction system in which a catalyst participating in reaction of a specific molecule is immobilized in the molecular imprinted gel of the present invention to allow the catalyst in the gel to specifically accelerate the reaction of the specific molecule upon shrinking of the gel due to the binding of the specific molecule to the gel.

Further, the molecular imprinted gel of the present invention can be used as a gel for the purification of a specific molecule and a gel for the analysis of biomolecular interactions based on its property to bind a specific molecule.

The gels of the present invention can be used in pharmaceutical compositions such as therapeutic agents, preventing agents and diagnostic agents for various diseases.

The pharmaceutical compositions of the present invention are preferably used for diseases induced by an increase of a causal substance. The pharmaceutical compositions may take, for example, the form of a molecular imprinted gel containing a pharmaceutically active ingredient which is prepared by causing the pharmaceutically active ingredient to be carried by the molecular imprinted gel prepared using a causal substance for a disease as a specific molecule and ligands therefor. The pharmaceutically active ingredient can be caused to be carried by the molecular imprinted gel by physical adsorption, chemical adsorption, etc. The physical adsorption includes adsorption by electrostatic interaction, hydrogen bond, hydrophobic interaction, etc. The chemical adsorption includes adsorption by covalent bond, etc.

When the molecular imprinted gel containing a pharmaceutically active ingredient is administered into a living organism, the gel shrinks at a diseased part where the specific molecule exists in excess, thereby causing the substance having the pharmaceutically active ingredient to be released from the gel and to act topically on the diseased part.

A specific example of the pharmaceutical compositions of the present invention is insulin-containing molecular imprinted gel as a therapeutic agent for diabetes. The insulin-containing molecular imprinted gel is produced by preparing a molecular imprinted gel using, as ligands, antibodies recognizing glucose which is the causal substance for diabetes, lectin recognizing sugar chain, etc., and then causing insulin to be carried within the gel. When administered into a living organism, the gel shrinks at a diseased part where glucose exists in excess, which causes insulin to be released from the gel.

For using the molecular imprinted gel as a pharmaceutical composition or an artificial organ, it is preferred to employ gel materials which do not induce rejection in vivo.

The pharmaceutical compositions such as therapeutic agents and preventing agents comprising the gels of the present invention may be administered as such to humans or animals, or may be mixed with one or more pharmaceutically acceptable carriers and provided as pharmaceutical preparations produced by any of the methods well known in the technical field of pharmaceutics.

It is desirable to administer the pharmaceutical composition by the route that is most effective for the treatment. Suitable administration routes include oral administration and parenteral administration such as intra-oral cavity administration, intratracheal administration, intrarectal administration, subcutaneous administration, intramuscular administration and intravenous administration.

The pharmaceutical composition may be in the form of spray, capsules, tablets, granules, syrup, emulsion, suppository, injection, ointment, tape, and the like.

The dose and administration schedule will vary depending on the desired therapeutic effect, the administration route, the period of treatment, the patient's age and body weight, etc. However, an appropriate daily dose of an active ingredient in the composition for an adult person is generally 10 µg/kg to 8 mg/kg.

Brief Description of the Drawings

Fig. 1 is a scheme showing a process for synthesizing a Con. A-introduced vinyl monomer.
Fig. 2 is a scheme showing a process for synthesizing a Con. A-introduced vinyl polymer.
Fig. 3 is a scheme showing a process for synthesizing an anti-AFP antibody-introduced vinyl monomer.
Fig. 4 is a scheme showing a process for synthesizing an AFP-imprinted gel.
Fig. 5 is a graph showing the change with the passage of time in the swelling ratio of an AFP-imprinted gel, an AFP-nonimprinted gel and a polyacrylamide (PAAm) gel in AFP-containing phosphate buffer after swelling attained equilibrium in phosphate buffer. ○, ● and □ indicate the results obtained with the AFP-imprinted gel, the AFP-nonimprinted gel and the PAAm gel, respectively.
Fig. 6 is a graph showing the relationship between the swelling ratio of an AFP-imprinted gel, an AFP-nonimprinted gel and a PAAm gel in AFP-containing phosphate buffer and the AFP concentration after swelling attained equilibrium in phosphate buffer. ○, ● and □ indicate the results obtained with the AFP-imprinted gel, the AFP-nonimprinted gel and the PAAm gel, respectively.
Fig. 7 is a graph showing the specific responsiveness of an AFP-imprinted gel by its reactivity to BSA and AFP. ○ and ● indicate the results obtained with AFP and BSA, respectively.
Fig. 8 is a graph showing the specific responsiveness of an AFP-imprinted gel by its reactivity to chicken ovalbumin and AFP. ○ and ● indicate the results obtained with AFP and chicken ovalbumin, respectively.

Example 1 Production of AFP-Imprinted Gel

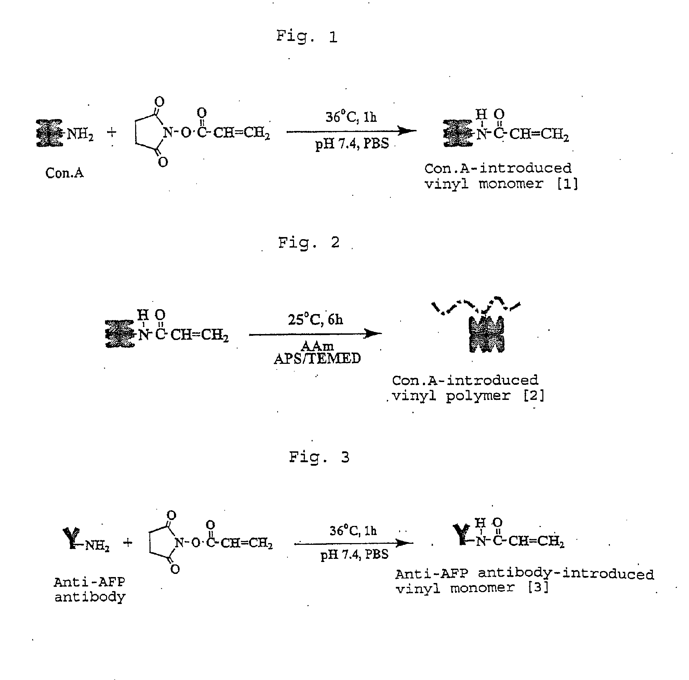

(1) Synthesis of Con. A-Introduced Vinyl Monomer (Fig. 1)

Con. A (20.4 mg, 0.2 mmol) was dissolved in 1 ml of 20 mmol/l phosphate-buffered saline (PBS) (pH 7.4, 25°C), and 0.03 ml (0.4 mmol) of a 2 mg/ml solution of N-succinimidyl acrylate (NSA) in dimethyl sulfoxide (DMSO) was added thereto, followed by stirring at 36°C for one hour.

The resulting reaction mixture was purified using column PD-10 (Pharmacia Biotech) packed with desalted Sephadex G-25 (Pharmacia Biotech) to obtain a solution containing a Con. A-introduced vinyl monomer. The ultraviolet absorption spectrum of the obtained solution containing the Con. A-introduced vinyl monomer was measured with a spectrophotometer (UV-2500PC, Shimadzu Corporation) to confirm that the solution containing the Con. A-introduced vinyl monomer had a characteristic absorption wavelength of 280 nm, which was the same as that of natural Con. A. Then, the concentration of the Con. A-introduced vinyl monomer in the solution containing it was determined from the calibration curve previously prepared using natural Con. A, whereby the concentration was found to be 4.3 mg/ml.

(2)Polymerization of Con. A-Introduced Vinyl Monomer (Fig. 2)

To 2 ml of the 4.3 mg/ml solution of Con. A-introduced vinyl monomer prepared in the above (1) were added 12 mg of acrylamide (AAm), 0.02 ml of a 0.1 mol/l aqueous solution of ammonium persulfate (APS) and 0.02 ml of a 0.8 mol/l aqueous solution of N, N,N',N'-tetramethylethylenediamine (TEMED). The resulting mixture was subjected to reaction at 25°C for 3 hours to obtain a solution containing a Con. A-introduced vinyl polymer.

(3) Synthesis of Anti-AFP Antibody-Introduced Vinyl Monomer (Fig. 3)

An anti-AFP antibody-introduced vinyl monomer was synthesized by a method similar to the above (1).
To 1 ml of a 2.2 mg/ml solution of rabbit polyclonal anti-human AFP antibody (Dako Japan Co., Ltd.) was added 0.01 ml of a 0.5 mg/ml solution of NSA in DMSO, followed by stirring at 36°C for one hour. The resulting reaction mixture was purified using column PD-10 (Pharmacia Biotech) packed with desalted Sephadex G-25 (Pharmacia Biotech). The fractions containing the anti-AFP antibody-introduced vinyl monomer were pooled and the obtained aqueous solution containing the anti-AFP antibody-introduced vinyl monomer was concentrated using a freeze dryer (FD-SN, Tokyo Rikakikai Co., Ltd.) to obtain a 185 mg/ml solution of the anti-AFP antibody-introduced vinyl monomer in phosphate buffer.

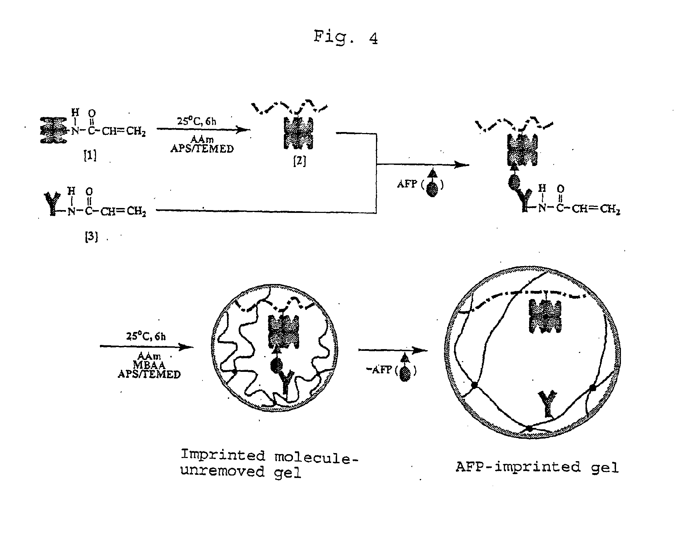

(4) Preparation of AFP-Imprinted Gel (Fig. 4)

An AFP-imprinted gel was prepared in the following manner according to the steps shown in Fig. 4. That is, 0.37 ml of a 0.11 mg/ml aqueous solution of AFP was added to a mixture of 0.012 ml of the solution of the Con. A-introduced vinyl polymer in phosphate buffer prepared in the above (2) and 0.2 ml of the 185 mg/ml solution of the anti-AFP antibody-introduced vinyl monomer in phosphate buffer prepared in the above (3). To the resulting solution were added 120 mg of AAm, 0.06 ml of a 1 mg/ml aqueous solution of N,N'-methylenebisacrylamide, 0.02 ml of a 0.1 mol/l aqueous solution of APS and 0.02 ml of a 0.8 mol/l aqueous solution of TEMED. The resulting solution was poured into a glass tube with one end closed (inner diameter: 3 mm) and subjected to reaction at 25°C for 6 hours. The gel obtained by the reaction was washed with phosphate buffer (pH 7.4) to remove the unreacted materials, and then washed with phosphate-citrate buffer (pH 4.0) to remove AFP, whereby the AFP-imprinted gel was obtained.

Example 2 Production of AFP-Nonimprinted Gel

A nonimprinted gel was prepared in the following manner according to a method similar to that described in Example 1 (4) without utilizing molecular imprinting. That is, 0.40 ml of PBS, instead of AFP, was added to a mixture of 0.012 ml of the solution of the Con. A-introduced vinyl polymer in phosphate buffer prepared in Example 1 (2) and 0.2 ml of the 185 mg/ml solution of the anti-AFP antibody-introduced vinyl monomer in phosphate buffer prepared in Example 1 (3). To the resulting solution were added 120 mg of AAm, 0.06 ml of a 1 mg/ml aqueous solution of N,N'-methylenebisacrylamide, 0.02 ml of a 0.1 mol/l aqueous solution of APS and 0.02 ml of a 0.8 mol/l aqueous solution of TEMED. The resulting solution was poured into a glass tube with one end closed (inner diameter: 3 mm) and subjected to reaction at 25°C for 3 hours. The gel obtained by the reaction was washed with phosphate buffer (pH 7.4), whereby the AFP-nonimprinted gel was obtained.

Comparative Example Preparation of PAAm Gel

A PAAm gel was prepared in the following manner according to a method similar to that described in Example 1 (4). To 0.62 ml of PBS were added 120 mg of AAm, 0.06 ml of a 1 mg/ml aqueous solution of N,N'-methylenebisacrylamide, 0.02 ml of a 0.1 mol/l aqueous solution of APS and 0.02 ml of a 0.8 mol/l aqueous solution of TEMED. The resulting solution was poured into a glass tube with one end closed (inner diameter: 3 mm) and subjected to reaction at 25°C for 3 hours, whereby the PAAm gel was prepared.

Example 3 Measurement of AFP in a Sample by Measurement of Swelling Ratio of Gels (1)

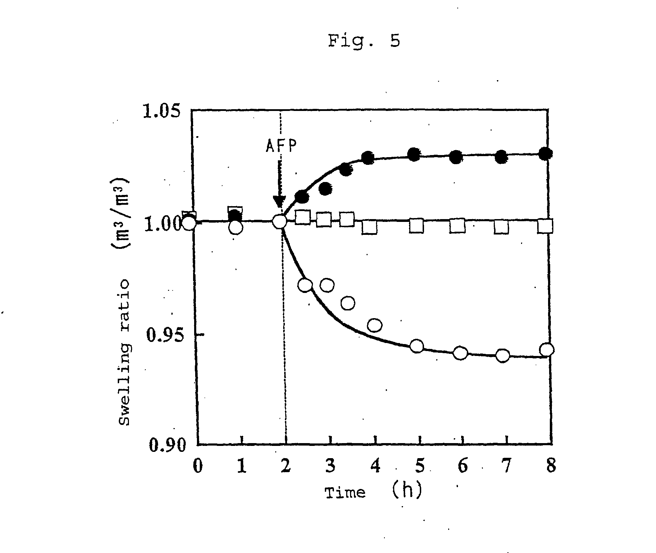

The change with the passage of time in the swelling ratio of the AFP-imprinted gel prepared in Example 1, the AFP-nonimprinted gel prepared in Example 2 and the PAAm gel prepared in Comparative Example upon exposure to AFP was observed (Fig. 5).
Each of the AFP-imprinted gel, AFP-nonimprinted gel and PAAm gel which had completely reached equilibrium swelling in phosphate buffer (pH 7.4, 0.02 mol/l) was added to a 40 µg/ml solution of AFP in phosphate buffer (pH 7.4, 0.02 mol/l) to subject the gel to an exposure to AFP. The volume change of the gel was evaluated as the swelling ratio of the gel. The swelling ratio of the gel was calculated by the following equation.

$$\text{Swelling ratio} = (d/d_0)^3$$

In the equation, $d_0$ means the diameter of the gel in the phosphate buffer, and d means the diameter of the gel in the AFP solution. If the value of swelling ratio is higher than 1, it means that the gel swelled; and if the value of swelling ratio is lower than 1, it means that the gel shrank.
The swelling ratio of the PAAm gel did not substantially change in the AFP solution. On the other hand, the AFP-nonimprinted gel swelled, and the AFP-imprinted gel shrank. This result indicates that qualitative analysis of AFP in a sample can be made by use of the AFP-imprinted gel and AFP-nonimprinted gel of the present invention.

Example 4 Measurement of AFP in a Sample by Measurement of Swelling Ratio of Gels (2)

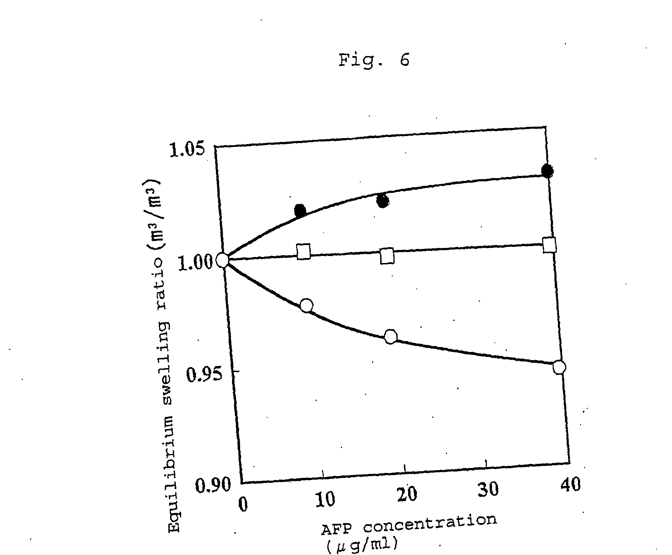

The effect of the AFP concentration on the swelling ratio of the AFP-imprinted gel prepared in Example 1, the AFP-nonimprinted gel prepared in Example 2 and the PAAm gel prepared in Comparative Example was examined (Fig. 6).
Each of the AFP-imprinted gel, AFP-nonimprinted gel and PAAm gel which had completely reached equilibrium swelling in phosphate buffer (pH 7.4, 0.02 mol/l) was added to solutions of AFP in phosphate buffer (pH 7.4, 0.02 mol/l) of varying concentrations. After the gel reached equilibrium swelling again, the swelling ratio of the gel was measured.
The swelling ratio of the PAAm gel was approximately constant independently of the AFP concentration. On the other hand, the AFP-nonimprinted gel swelled in a manner dependent on the AFP concentration, and the AFP-imprinted gel shrank in a manner dependent on the AFP concentration. The concentration of AFP in a sample can be determined using the AFP-imprinted gel or AFP-nonimprinted gel by using, as a calibration curve, the relationship between the swelling ratio of the gel in the AFP solution and the AFP concentration in the solution as shown in Fig. 6.

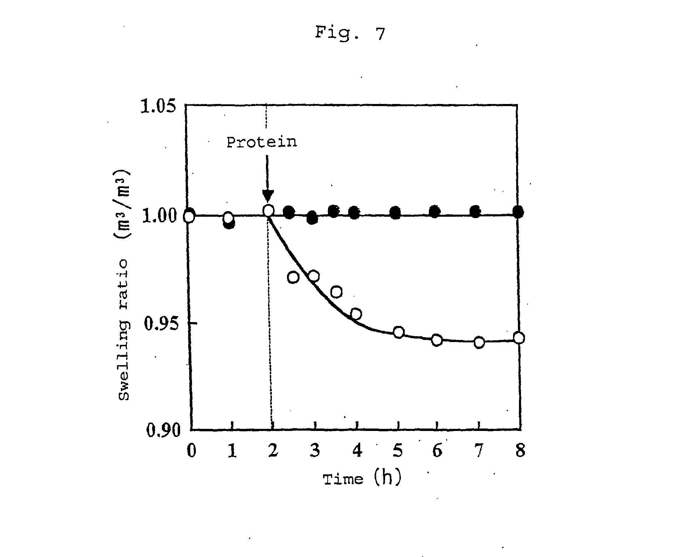

Reference Example 1 Specific Responsiveness of AFP-Imprinted Gel (1) (Fig. 7)

The specific responsiveness of an AFP-imprinted gel was examined using the AFP-imprinted gel which had completely reached equilibrium swelling in phosphate buffer (pH 7.4, 0.02 mol/l). The AFP-imprinted gel was subjected to an exposure to BSA, which is a substance recognized by neither Con. A nor an anti-AFP antibody.

When added to a 40 µg/ml solution of BSA in phosphate buffer (pH 7.4, 0.02 mol/l), the AFP-imprinted gel neither shrank nor swelled as shown in Fig. 7. On the other hand, the gel shrank when added to a solution of AFP in phosphate buffer (pH 7.4, 0.02 mol/l). This result indicates that the AFP-imprinted gel shrinks in response to AFP recognizing both a sugar chain and an anti-AFP antibody, but undergoes no volume change in response to BSA recognizing neither a sugar chain nor an anti-AFP antibody.

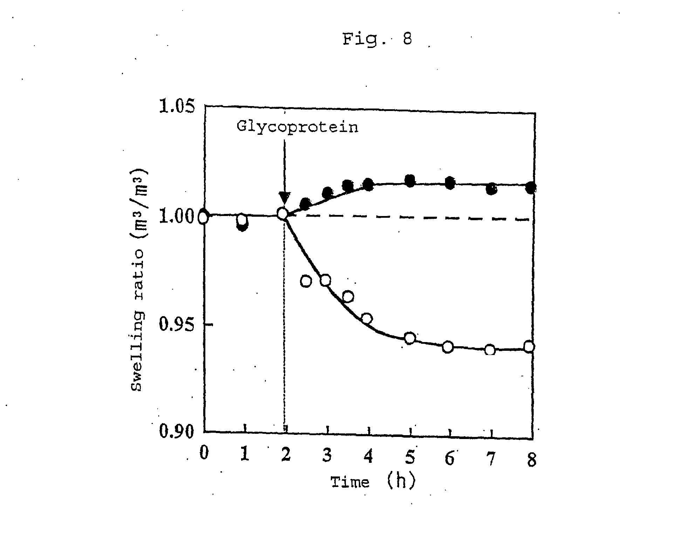

Reference Example 2 Specific Responsiveness of AFP-Imprinted Gel (2) (Fig. 8)

The specific responsiveness of an AFP-imprinted gel was examined using chicken ovalbumin instead of BSA used in Reference Example 1. Chicken ovalbumin is a substance recognized by Con. A, but not by an anti-AFP antibody.

When added to a 40 µg/ml solution of chicken ovalbumin in phosphate buffer (pH 7.4, 0.02 mol/l), the AFP-imprinted gel swelled as shown in Fig. 8. On the other hand, the gel shrank when added to a solution of AFP in phosphate buffer (pH 7.4, 0.02 mol/l). This result indicates that the AFP-imprinted gel shrinks in response to AFP recognizing both a sugar chain and an anti-AFP antibody, but swells in response to chicken ovalbumin recognizing only a sugar chain.

Industrial Applicability

The present invention provides a molecular imprinted gel, an imprinted molecule-unremoved gel and a nonimprinted gel in which two or more ligands which are biomacromolecules are grafted. The high specificity (specific molecule-recognizing ability) of these gels enables simple measurement of a specific molecule in a sample and simple diagnosis of various diseases.

**Claims**

1. A gel in which two or more ligands which are biomacromolecules are grafted.

2. The gel according to Claim 1 which undergoes a structural change induced by binding of a specific molecule which binds to the ligands.

3. The gel according to Claim 1 or 2 which is produced by molecular imprinting using a specific molecule as a template and in which two or more ligands for the specific molecule are grafted and the specific molecule is not contained.

4. The gel according to Claim 1 or 2 which is produced by molecular imprinting using a specific molecule as a template and in which two or more ligands for the specific molecule are grafted and the specific molecule is contained.

5. The gel according to Claim 1 or 2 which is produced without utilizing molecular imprinting using a specific molecule as a template and in which two or more ligands for the specific molecule are grafted.

6. The gel according to any one of Claims 1 to 3 which shrinks by the action of a specific molecule.

7. The gel according to any one of Claims 1, 2, 4 and 5 which swells by the action of a specific molecule.

8. The gel according to any one of Claims 1 to 7, wherein the biomacromolecule is selected from the group consisting of a protein, an antigen, an antibody, the complementarity determining region of an antibody, lectin, DNA, RNA, a lipid, a polysaccharide and a complex thereof.

9. A process for producing the gel according to Claim 3 which comprises the step of chemically immobilizing in a gel two or more ligands for a specific molecule which are bound to the specific molecule as a template, and the step of removing the specific molecule from the gel.

10. A process for producing the gel according to Claim 4 which comprises the step of chemically immobilizing in a gel two or more ligands for a specific molecule which are bound to the specific molecule as a template.

11. A process for producing the gel according to Claim 5 which comprises the step of chemically immobilizing in a gel two or more ligands for a specific molecule without using the specific molecule as a template.

12. A method for measuring a specific molecule in a sample which is **characterized by** use of the gel according to any one of Claims 1 to 8.

13. An apparatus comprising the gel according to any one of Claims 1 to 8.

14. A measurement kit comprising the gel according to any one of Claims 1 to 8.

15. A pharmaceutical composition comprising the gel according to any one of Claims 1 to 8.

**EP 1 391 730 A1**

**16.** A diagnostic method for various diseases using the measurement kit according to Claim 14.

Fig. 1

Con.A-introduced
vinyl monomer [1]

Fig. 2

Con.A-introduced
vinyl polymer [2]

Fig. 3

Anti-AFP antibody-introduced
vinyl monomer [3]

Fig. 4

Imprinted molecule-
unremoved gel

AFP-imprinted gel

Fig. 5

Fig. 6

Fig. 7

Fig. 8

## INTERNATIONAL SEARCH REPORT

| | International application No. |
|---|---|
| | PCT/JP02/04332 |

**A. CLASSIFICATION OF SUBJECT MATTER**
Int.Cl$^7$  G01N33/544, A61M1/36, A61K9/00, A61K38/28

According to International Patent Classification (IPC) or to both national classification and IPC

**B. FIELDS SEARCHED**

Minimum documentation searched (classification system followed by classification symbols)
Int.Cl$^7$  G01N33/544, A61M1/36, A61K9/00, A61K38/28

Documentation searched other than minimum documentation to the extent that such documents are included in the fields searched
Jitsuyo Shinan Koho          1922-1996   Toroku Jitsuyo Shinan Koho   1994-2002
Kokai Jitsuyo Shinan Koho    1971-2002   Jitsuyo Shinan Toroku Koho   1996-2002

Electronic data base consulted during the international search (name of data base and, where practicable, search terms used)
JOIS, BIOSIS

**C. DOCUMENTS CONSIDERED TO BE RELEVANT**

| Category* | Citation of document, with indication, where appropriate, of the relevant passages | Relevant to claim No. |
|---|---|---|
| Y | Nature, Vol.399, (1999), pages 766 to 769 | 1-15 |
| Y | Macromolecules, Vol.32, No.6, (1999), pages 2082 to 2084 | 1-15 |
| Y | JP 1-265972 A  (Kaneka Corp.), 24 October, 1989 (24.10.89), (Family: none) | 1,5,8, 11-14 |
| Y | JP 7-118340 A  (Nippon Zeon Co., Ltd.), 09 May, 1995 (09.05.95), (Family: none) | 1,5,8, 11-14 |
| A | JP 2000-88827 A  (Koki RI), 31 March, 2000 (31.03.00), (Family: none) | 1-15 |

☐ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 10 July, 2002 (10.07.02) | 23 July, 2002 (23.07.02) |

| Name and mailing address of the ISA/ | Authorized officer |
|---|---|
| Japanese Patent Office | |
| Facsimile No. | Telephone No. |

Form PCT/ISA/210 (second sheet) (July 1998)

## INTERNATIONAL SEARCH REPORT

International application No.

PCT/JP02/04332

**Box I    Observations where certain claims were found unsearchable (Continuation of item 1 of first sheet)**

This international search report has not been established in respect of certain claims under Article 17(2)(a) for the following reasons:

1. [X] Claims Nos.: 16

   because they relate to subject matter not required to be searched by this Authority, namely:

   It pertains to diagnostic methods.

2. [ ] Claims Nos.:

   because they relate to parts of the international application that do not comply with the prescribed requirements to such an extent that no meaningful international search can be carried out, specifically:

3. [ ] Claims Nos.:

   because they are dependent claims and are not drafted in accordance with the second and third sentences of Rule 6.4(a).

**Box II    Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**    [ ]    The additional search fees were accompanied by the applicant's protest.

[ ]    No protest accompanied the payment of additional search fees.

Form PCT/ISA/210 (continuation of first sheet (1)) (July 1998)